(19) Europäisches Patentamt
European Patent Office
Office européen des brevets

(11) Numéro de publication : **0 155 885**
**B1**

(12) # FASCICULE DE BREVET EUROPÉEN

(45) Date de publication du fascicule du brevet : **09.11.88**

(21) Numéro de dépôt : **85400395.1**

(22) Date de dépôt : **01.03.85**

(51) Int. Cl.[4] : **C 07 C 47/14**, C 07 C 45/63, C 07 C 47/24, C 07 C 45/51, C 07 C 41/50, C 07 C 41/30, C 07 C 43/313, C 07 C 43/192, C 07 C 51/58, C 07 C 53/50, C 07 C 69/63

(54) **Dihalogéno-2-3-fluoro-pronanals, leur procédé d'obtention et leurs applications à l'obtention d'halogénures et d'esters de dihalogéno-2,3-fluoro-2-propanoyles et de fluoroacrylates d'alkyle ou d'aryle.**

(30) Priorité : **02.03.84 FR 8403290**
**05.03.84 FR 8403376**

(43) Date de publication de la demande :
**25.09.85 Bulletin 85/39**

(45) Mention de la délivrance du brevet :
**09.11.88 Bulletin 88/45**

(84) Etats contractants désignés :
**DE GB IT NL**

(56) Documents cités :
**EP-A- 0 093 255**
**US-A- 2 351 000**
**US-A- 2 454 663**
**US-A- 2 815 384**
**CHEMICAL ABSTRACTS, Band 81, Nr. 15, 14. Oktober 1974, Seite 419, Nr. 91004r, Columbus, Ohio, US; B.L. DYATKIN u.a.: "Alpha-fluoro-beta-haloacrylic acids"**
**JUSTUS LIEBIGS ANNALEN DER CHEMIE, vol. 710, 1967, pages 36-58,Verlag Chemie GmbH, Weinheim, DE; F. NERDEL u.a.: "Reaktionen von Dihalogencarbenen mit Enoläthern"**
**TETRAHEDRON LETTERS, no. 44, 1966, pages 5379-5383, Pergamon Press Ltd., Londres, GB; J. BUDDRUS u.a.: "Darstellung von 2-Fluor-Acrolein"**
**HOUBEN-WEYL: "Methoden der organischen Chemie", vol. VIII, Sauerstoffverbindungen III, 1952, page 474, Georg Thieme Verlag, Stuttgart, DE;**

(73) Titulaire : **INSTITUT NATIONAL DE RECHERCHE CHIMIQUE APPLIQUEE**
**18 Bis, Boulevard de la Bastille**
**F-75012 Paris (FR)**

(72) Inventeur : **Lampin, Jean-Pierre**

**décédé (FR)**
Inventeur : **Nonat, Alain**
**Rue Lavoisier**
**F-91710 Vert-le-Petit (FR)**
Inventeur : **Vignal, Guy**
**Ecole de Vert-le-Petit**
**F-91170 Vert-le-Petit (FR)**
Inventeur : **Wakselman, Claude**
**99 Rue de Paris**
**F-91400 Orsay (FR)**
Inventeur : **Molines, Huguette**
**10, Rue des Jardiniers**
**F-75012 Paris (FR)**
Inventeur : **Nguyen, Thoai**
**21, Rue de Verdun**
**F-94260 Fresnes (FR)**

(74) Mandataire : **Rinuy, Santarelli**
**14, avenue de la Grande Armée**
**F-75017 Paris (FR)**

**EP 0 155 885 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux composés dihalogéno-2,3-fluoro-2-propanals de formule :

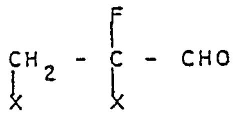

avec x = Cl ou Br (I)

Ces composés s'avèrent particulièrement intéressants car ils permettent d'accéder par voie de synthèse aux fluoroacrylates d'alkyle par voie de synthèse aux fluoroacrylates d'alkyle ou d'aryle, lesquels peuvent servir de matière de départ pour l'obtention de polymères acryliques possédant des caractéristiques améliorées.

L'examen de la formule des composés selon l'invention fait apparaître une particularité, à savoir qu'ils ne présentent pas d'atome d'hydrogène en position alpha par rapport à la fonction carbonyle.

Il en résulte qu'une halogénation se traduit par une substitution du proton aldéhydique par l'halogène, d'où la possibilité d'obtenir l'halogénure d'acide correspondant, lequel permet à son tour d'accéder aux acrylates.

Les demandeurs ont trouvé que les composés selon l'invention peuvent être obtenus, entre autres, soit à partir de fluoro-2-acroléine de formule :

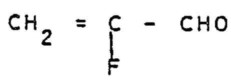

soit de ses précurseurs et ils ont mis au point un procédé nouveau et économique à l'effet d'obtenir de bons rendements en fluoro-2-acroléine.

En effet à l'heure actuelle, on ne connaît qu'une seule synthèse de la fluoro-2-acroléine. Il s'agit d'une synthèse de laboratoire. Elle a été présentée par J. Buddrus et al. — Tetrahedron Letters 1966 (44), 5379-83 et Justus Liebig — Ann. Chem., 1967, 710, 36-58.

Selon ces auteurs, on traite en autoclave à 150 °C un mélange de butylvinyl éther, d'oxyde d'éthylène et de dichlorofluorométhane en présence de bromure de tétraéthylammonium. Le produit de réaction est le butoxy-3-(chloro-2-éthoxy)3-fluoro-2-propène :

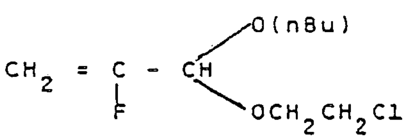

(II)

Ces mêmes auteurs suggèrent pour cette réaction un mécanisme passant par un dérivé de cyclopropane de formule :

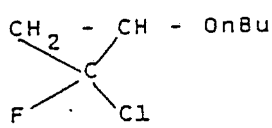

non isolé, lequel s'ouvrirait dans les conditions de l'essai pour donner, en réagissant avec l'oxyde d'éthylène, l'acétal II.

L'hydrolyse de II en milieu sulfurique conduit à la fluoro-acroléine qui est isolée par distillation. Le rendement annoncé par ces auteurs, sur l'ensemble des deux étapes est de 21,7 %.

Si l'on tente d'appliquer cette synthèse à l'échelle industrielle, l'exothermicité de la réaction la rend incontrôlable quand on fait appel à des quantités de réactif de l'ordre ou supérieures à la mole ; il se développe brutalement en effet des pressions supérieures à 300 bars, rendant difficile toute extrapolation.

Or les demandeurs obvient à ces inconvénients en fournissant un procédé ne présentant pas ces risques, ce procédé pouvant être conduit sans nécessiter d'autoclave en faisant appel à des réacteurs connus d'usage courant. De plus, ce procédé ne fait appel à de l'oxyde d'éthylène qui est une substance toujours très délicate à mettre en œuvre en quantités importantes.

Ce procédé consiste à effectuer l'hydrolyse d'acétals de formule :

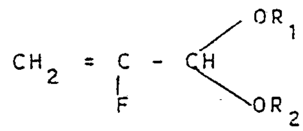

dans laquelle $R_1$ et $R_2$ représentent des groupes alkyles identiques ou différents ayant de 1 à 8 atomes de carbone.

De façon plus particulière, les acétals de départ sont avantageusement obtenus par ouverture dans des conditions douces et contrôlables de cycles d'alcoxy-1-halogéno-2-fluoro-2-cyclopropanes de formule :

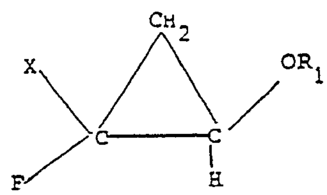

où $R_1$ est un alkyle de $C_1$ à $C_8$ et X est Cl ou Br par solvolyse en milieu alcoolique ($R_2OH$) en présence d'une base, ces cycles cyclopropaniques étant eux-mêmes obtenus à partir d'un éther énolique de formule :

$$CH_2 = CH—OR_1$$

avec $R_1$ = alkyle de $C_1$ à $C_8$, par addition de halogénofluorocarbène. De façon avantageuse, on fera intervenir le chlorofluorocarbène lui-même obtenu à partir de dichlorofluorométhane ($CHFCl_2$) (« Fréon 21 »), en présence d'une base et d'un agent de transfert de phase.

Le mode de mise en œuvre du procédé tel que défini ci-dessus peut alors être schématisé par la série de réactions suivantes :

a) transformation d'un alkylvinyl éther de formule :

$$CH_2 = CH—OR_1 \qquad (III)$$

où $R_1$ = alkyle de $C_1$ à $C_8$, en alcoxy-2-chloro-1-fluoro-1-cyclopropane par addition de chlorofluorocarbène (CFCl). On obtient ainsi un mélange des deux isomères syn et anti de formule :

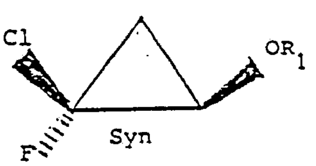


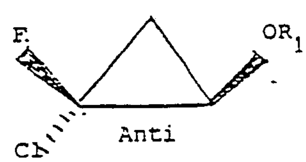


Le chlorofluorocarbène est lui-même obtenu in situ à partir du dichlorofluorométhane ($CHFCl_2$) (« Fréon 21 ») en milieu alcalin (NaOH ou KOH) en présence d'un catalyseur de transfert de phase du type bromure de tétraéthylammonium (TEBA) et analogues ;

b) conversion de l'alcoxy-2-chloro-1-fluoro-1-cyclopropane (IV) en dialcoxy-3,3-fluoro-2-propène (V) par action d'une base en milieu alcoolique suivant :

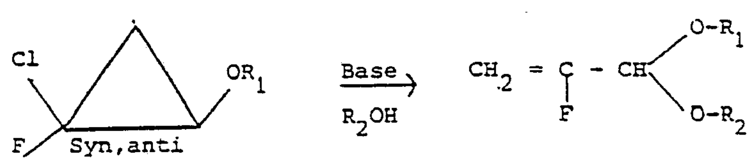


(IV)

avec $R_1$ et $R_2$ identiques ou différents = alkyle ou aryle en $C_1$ à $C_8$.

Parmi les bases pouvant être utilisées, on peut citer en particulier la pyridine, la triéthanolamine, la soude et analogues. Cette réaction est avantageusement menée à une température de l'ordre de 110/120 °C ;

c) hydrolyse de l'acétal (V) par un acide minéral tel que HCl ou $H_2SO_4$, pour obtenir, la fluoro-2-acroléine suivant :

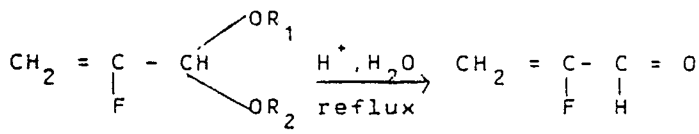


(V)

Les rendements que l'on peut obtenir en mettant en œuvre ce procédé sont de l'ordre de 60 % et plus, calculé sur la base de l'alkylvinyl éther de départ, alors que dans le procédé connu, à l'autoclave, le rendement est seulement de 21,7 %.

Disposant ainsi d'un moyen conduisant aisément à la fluoro-2-acroléine, les composés selon l'invention peuvent être obtenus directement à partir de la fluoro-2-acroléine, par addition d'un équivalent d'halogène ($Cl_2$ ou $Br_2$), à froid, selon la réaction :

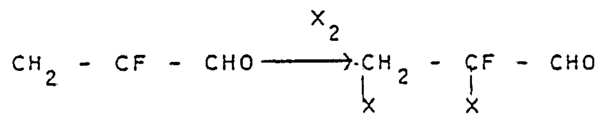


avec X = Cl ou Br.

Les dihalogénures peuvent être ensuite isolés par distillation sous pression réduite.

Ou bien, ils peuvent être obtenus à partir de l'éther d'énol correspondant, matière première servant à l'élaboration de la fluoro-2-acroléine, sans qu'il soit nécessaire d'isoler ladite fluoroacroléine à l'état pur.

Le processus peut alors s'énoncer ainsi :

$CH_2 = CH—OR_1$ $R_1$ = alkyle de $C_1$ à $C_8$

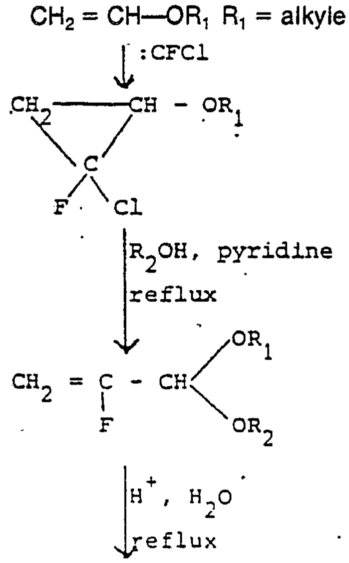


$CH_2 = CF—CHO$, ($R_1OH$, $R_2OH$, $H_2O$).

A l'issue de cette dernière étape, le milieu réactionnel est composé de fluoro-2-acroléine, d'eau, et des alcools $R_1OH$ et $R_2OH$. Un séchage grossier sur $CaCl_2$ suivi d'une simple décantation donne un mélange qu'il est possible de bromer directement à froid et qui conduit au dibromo-2,3-fluoro-2-propanal, que l'on pourra isoler selon les techniques habituelles.

Comme indiqué précédemment, la particularité des dihalogéno-2,3-fluoro-2-propanals est que l'on est en présence d'aldéhydes ne possédant pas d'atomes d'hydrogène en position alpha par rapport à la fonction carbonyle.

Une halogénation plus poussée va donc conduire à l'halogénure d'acide correspondant, c'est-à-dire à l'halogénure de dihalogéno-2,3-fluoro-2-propanoyle. Cette halogénation peut se faire par action du brome à chaud, sous irradiation ultra-violette ou par action du chlore à température ambiante.

On peut ainsi utiliser d'autres agents halogénants, tels que le N-bromo-succinimide par exemple. Ces halogénations pourront s'effectuer en présence d'initiateurs de radicaux libres, tels le peroxyde de benzoyle et autres.

A partir de l'halogénure d'acide précité, l'accès aux fluoro-2-acrylates d'alkyle ou d'aryle est relativement aisé.

L'halogénure d'acide dihalogéné soumis à l'action d'un alcool ou d'un phénol en présence de base (pyridine ou triéthylamine $Et_3—N$) conduit au dihalogéno-2,3-fluoro-2-propionate d'alkyle ou d'aryle.

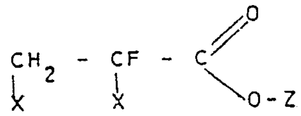

où Z = alkyle ou aryle
et X = chlore ou brome

lequel est déshalogéné par le zinc pour finalement conduire au fluoro-2-acrylate d'alkyle ou d'aryle.

Les exemples suivants sont donnés à titre illustratif et nullement limitatif de la présente invention.

## Exemple 1

A) Formation du butoxy-1-chloro-2-fluoro-2-cyclopropane

Dans un réacteur à double enveloppe refroidi au glycol, muni d'un moyen d'agitation énergique, on introduit une solution constituée par 1,5 litre d'eau et 1 500 g de soude en paillettes. Ce mélange étant refroidi à 0 °C, on ajoute successivement 1 300 ml de chlorure de méthylène, 21,6 g de bromure de tétraéthylammonium et 701 g de butylvinyléther (7 moles).

A l'aide d'un dispositif permettant une introduction gazeuse au sein de la masse réactionnelle, on injecte 828 g de « Fréon 21 » (8 moles) tout en veillant à ce que la température n'excède pas 5 °C.

On peut toutefois s'assurer de la fin de réaction par l'absence de protons vinyliques en RMN$^1$H.

En fin de réaction, il est ajouté de l'eau jusqu'à démixtion des deux phases (6,5 litres).

Après décantation, on extrait la phase aqueuse par du chlorure de méthylène et on lave la phase organique par une solution saturée de chlorure de sodium. Le chlorure de méthylène est chassé. Après séchage par distillation azéotropique en présence de dichloroéthane, il reste un produit enrichi à 93 % en butoxy-1-chloro-2-fluoro-2-cyclopropane.

Après distillation sous pression réduite (16 mm Hg-55 °C), on obtient 926 g d'un produit à 98 % de pureté, soit un rendement de 78,1 % en produit pur.

Il s'agit d'un mélange des deux isomères syn et anti.

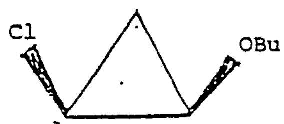

Syn
∿58 %

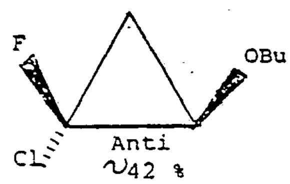

Anti
∿42 %

Caractérisation :

Caractérisation :
Eb = 55 ° C/16 mm Hg
RMN$^1$H = >CH— et —$OCH_2$— = motif centré à 3,68 ppm
—$CH_3$ = motif centré à 0,96 ppm
—$CH_2$— = motif centré à 1,49 ppm
RMN$^{19}$F = $CDCl_3/C_6F_6$
isomère syn = motif centré à 26,9 ppm
isomère anti = motif centré à 5,3 ppm.

Le bromure de tétraéthylammonium peut être remplacé par tout autre agent de transfert de phase, en particulier par le TEBA (chlorure de triéthylbenzylammonium) etc.

B) Formation de l'éthoxy-1-chloro-2-fluoro-2-cyclopropane

Un essai semblable à celui décrit en A) mais utilisant l'éthylvinyléther au lieu de butylvinyléther a conduit à l'éthoxy-1-chloro-2-fluoro-2-cyclopropane.

Caractérisation :
Rdt = 58 %
Eb = 112-114 °C/760 mm Hg
RMN$^1$H = >CH— et —$OCH_2$ = motif centré à 3,75 ppm
—$CH_3$ = motif centré à 1,27 ppm
—$CH_2$— = motif centré à 1,67 ppm
RMN$^{19}$F = $CDCl_3/C_6F_6$
isomère syn = motif centré à 25,5 ppm
isomère anti = motif centré à 3,83 ppm

C) Formation de l'isobutoxy-1-chloro-2-fluoro-2-cyclopropane.

On procède de la même manière en utilisant l'isobutylvinyléther.
Caractérisation :
Rdt = 82,1 %
Eb = 50-55 °C/18 mm Hg
RMN $^1H$ = >CH— et —$OCH_2$— = motif centré à 3,40 ppm
—$CH_2$— et >CH—
(isobutyl) = motif étalé de 1,33 à 1,90 ppm
—$CH_3$ = doublet (0,88-0,96) ppm
RMN $^{19}F$ = isomère syn à 27,1 ppm
isomère anti à 5,3 ppm.

Exemple 2

A) Formation du di-n-butoxy-1,1-fluoro-2-propène

Dans un tricol muni d'une ampoule de coulée et d'un dispositif de reflux, on introduit, en agitant, 102,5 g de nBuOH et 43,4 g de pyridine.

On porte à reflux, puis on coule 83 g de butoxy-1-chloro-2-fluoro-2-cyclopropane tel que préparé en 1A. Le reflux est maintenu deux heures et demi à trois heures. Après refroidissement, on introduit environ 90 ml d'eau jusqu'à dissolution du chlorhydrate de pyridine.

Après traitement du milieu réactionnel (décantations, lavages et extractions des différentes phases, évaporation des solvants), l'acétal est distillé.

On recueille 81,6 g d'un acétal à 98 % de pureté, soit un rendement de 78,4 % en produit pur.

Caractérisation :
Eb = 64-67 °C/4 mm Hg
RMN $^1H$ = —$CH_2$— et >CH— = motif centré à 4,77 ppm
$OCH_2$— = motif centré à 3,57 ppm
—$CH_2$— = motif centré à 1,52 ppm
$CH_3$— = motif centré à 0,92 ppm
RMN $^{19}F$ = $CDCl_3/C_6F_6$ = motif centré à 52,3 ppm.

B) Formation du di-isobutoxy-1,1-fluoro-2-propène

On procède comme décrit en A) ci-dessus mais en utilisant l'isobutoxy-1-chloro-2-fluoro-2-cyclopropane et l'isobutanol.

On obtient ainsi le di-isobutoxy-1,1-fluoro-2-propène caractérisé par :
Rdt = 77,2 %
Eb = 73-76 °C/4 mm Hg
RMN $^1H$ = —CH3 = doublet 0,89 à 0,97 ppm
—CH<(iBu) = multiplet centré à 1,87 ppm
$OCH_2$— = motif centré à 3,30 ppm
>$CH_2$— et
—CH<(cycle) = motif allant de 4,50 à 5,11 ppm.

C) Un essai identique à celui décrit en A) ci-dessus a été effectué en remplaçant la pyridine par la triéthanolamine.

L'évolution est beaucoup plus lente. Après 5 heures, une C.P.G. révèle que 20 % du produit de départ n'a pas encore réagi. Il faut maintenir le reflux une quinzaine d'heures pour que la totalité du dérivé cyclopropanique disparaisse.

Après traitement, l'acétal a pu être isolé avec un rendement de l'ordre de 50 %.

Exemple 3

A) Formation de la fluoroacroléine

Dans un réacteur de 2 litres équipé d'une tête de distillation à reflux total et d'un condenseur, on met 408 g de di-n-butoxy-1,1-fluoro-2-propène (2 moles) et 750 ml d'acide chlorhydrique N.

La tête de reflux étant fermée, on porte à reflux. La température de vapeur sur la tête de distillation qui est de 93 °C environ (azéotrope butanol-eau) descend lentement pour se stabiliser aux alentours de 77-78 °C, au bout d'une heure environ. A cet instant, on récupère le distillat dans la recette jusqu'à ce que la température se restabilise à nouveau vers 93 °C. On recueille ainsi 340 g environ d'un mélange ternaire : fluoro-2-acroléine, eau et butanol.

Le mélange est mis sur chlorure de calcium ; on le laisse décanter et puis on distille « bulbe à bulbe ». Cette suite d'opérations est répétée deux à trois fois consécutivement, selon la pureté du produit désirée.

On isole ainsi 123,4 g d'un produit à 97,6 % de pureté (C.P.G.), soit un rendement de 81,4 % en produit pur.

Caractérisation :

RMN[1]H = dans $CDCl_3$

proton aldéhyde : 2 pics à 9,26-9,48 ppm

proton vinylique : motif centré à 5,65 ppm

RMN[19]F = $CDCl_3/C_6F_6$ : motif centré à 41,8 ppm.

Exemple 4

Formation du dibromo-2,3-fluoro-2-propanal à partir de la fluoroacroléine

Dans un ballon de 500 ml muni d'une agitation magnétique, réfrigérant et ampoule de coulée, on introduit 111 g de fluoro-2-acroléine (1,5 mole) en solution dans 110 ml de tétrachlorure de carbone. Tout en maintenant la température vers 0 °C, on coule lentement 240 g de brome (1,5 mole). Après décoloration du milieu et évaporation du tétrachlorure de carbone, une chromatographie en phase aqueuse révèle que l'on a un produit relativement pur (95 %).

Ce produit peut être distillé sous pression réduite. On obtient ainsi :

m = 182 g, soit un rendement de l'ordre de 52 %.

Caractérisation :

Eb = 56-57 °C sous 19 mm Hg

RMN[1]H = proton aldéhyde = motif centré à 9,31 ppm

$-CH_2-$ = motif centré à 4,13 ppm

RMN[19]F = $CDCl_3/C_6F_6$ = motif centré à 34,30 ppm

Exemple 5

Formation du dichloro-2,3-fluoro-2-propanal à partir de la fluoroacroléine

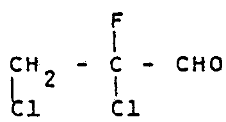

On procède comme dans l'exemple 4, à froid, (— 10 °C à — 15 °C), en présence de chlore.

Caractérisation :

Eb = 110-113 °C/760 mm Hg

RMN[1]H = $CH_2$ = 4,03 et 4,3 ppm

CH— = 9,55 ppm

RMN[19]F = $CDCl_3/C_6F_6$ : motif à 31 ppm.

Exemple 6

Formation du bromure de dibromo-2,3-fluoro-2-propanoyle

A) A partir du dibromo-2,3-fluoro-2-propanal

A 0,05 mole de dibromo-2,3-fluoro-2-propanal en solution dans 37 ml de $CCl_4$, on ajoute, rapidement, à froid, 80 g de brome ainsi que 0,2 g de peroxyde de benzoyle. On porte alors à reflux en même temps que l'on irradie sous ultraviolets. Des prélèvements périodiques soumis à la C.P.G permettent de suivre l'évolution de la réaction que l'on arrête après disparition du produit de départ.

Après évaporation de l'excès de brome et des solvants, on a un produit relativement pur que l'on peut utiliser tel quel.

Une distillation sous pression réduite permet une purification supplémentaire : m = 117,3 g, pureté plus ou moins 90 %, soit un rendement en produit pur de l'ordre de 67,5 %.

Caractérisation :

Eb = 54 °C/6 mm Hg

RMN[1]H = motif centré à 4,30 ppm (4 pics : 4,52-4,38 4,21-4,07 ppm)

RMN[19]F = $CDCl_3/C_6F_6$ : motif centré à 54,57 ppm.

B) A partir de la fluoroacroléine

Dans un ballon de 250 ml muni d'une agitation, un réfrigérant et une ampoule de coulée, on introduit 37 g de fluoro-2-acroléine (0,5 mole) en solution dans 37 ml de $CCl_4$. Tandis que le mélange est refroidi à — 5 °C, 0 °C, on coule lentement, par l'intermédiaire de l'ampoule de coulée, 79 g de brome. On observe

la décoloration au fur et à mesure de l'addition.

En fin d'addition, toujours à froid, on verse rapidement 80 g de brome dans le mélange, puis on porte à reflux en même temps que l'on irradie sous ultraviolets.

Après trois heures de reflux, on vérifie que toute la fluoroacroléine a été consommée. Une distillation sous pression réduite (6 mm-54 °C) permet d'isoler 116,7 g de bromure d'acide à 89 % de pureté, soit un rendement en produit pur de 66,6 %.

Exemple 7

Formation du chlorure de dichloro-2,3-fluoro-2-propanoyle

On fait barboter un courant de chlore dans une solution constituée par 2,9 g de dichloro-2,3-fluoro-2-propanal en solution dans du chlorure de méthylène, et ce, à une température de — 15 °C, — 10 °C.

On arrête le barbotage lorsqu'un prélèvement soumis à un spectre $RMN^{19}F$ montre la disparition de la raie correspondant à l'aldéhyde (31 ppm).

Après évaporation des solvants et distillation, on obtient le chlorure de dichloro-2,3-fluoro-2-propanoyle :

Caractérisation :
Eb = 100-108 °C/760 mm
$RMN^1H$ = motif de 4,1 à 4,7 ppm
$RMN^{19}F$ = motif à 47 ppm
Rdt = 30 %

Exemple 8

Formation du dibromo-2,3-fluoro-2-propionate de phényle

A) A partir du phénate de potassium

Dans un réacteur d'un litre, sous atmosphère d'argon, avec agitation et réfrigérant, on introduit 150 g de bromure de dibromo-2,3-fluoro-2-propanoyle (teneur 69 %, soit 0,33 mole) dans 480 ml de $CH_2Cl_2$. Le mélange étant maintenu à 0 °C, on ajoute 63 g de phénate de potassium fraîchement préparé. Après maintien de l'agitation pendant une heure à 0 °C, on laisse revenir à température ambiante.

Après addition de 120 ml d'eau et décantation, la phase aqueuse est extraite par du chlorure de méthylène.

L'ensemble des phases organiques est lavé par un mélange eau-carbonate acide de sodium puis distillé.

La fraction 105-115 °C/0,7 mm Hg permet d'isoler 85,7 g de dibromo-2,3-fluoro-2-propionate de phényle, soit un rendement de l'ordre de 79,6 %.

Caractérisation :
Eb = 105-115 °C/0,7 mm Hg
$RMN^1H$ = $-CH_2-$ = motif centré de 4,09 à 4,56 ppm
phényl = motif centré à 7,3 ppm
$RMN^{19}F$ = motif centré à 43,63 ppm.

B) A partir du phénol en présence de pyridine

Dans un ballon de 2 litres muni d'une agitation, un réfrigérant et une ampoule de coulée, on introduit 66,7 g de phénol (0,75 mole) en solution dans 750 ml de tétrachlorure de carbone. Au moyen de l'ampoule de coulée, on ajoute encore 59,2 g de pyridine (0,75 mole).

L'ensemble étant maintenu sur bain réfrigérant de sorte que la température n'excède pas 20 °C, on ajoute le bromure d'acide brut (bromure de dibromo-2,3-fluoro-2-propanoyle) issu de la bromation de 0,75 mole de fluoro-2-acroléine, et ce lentement, par l'intermédiaire de l'ampoule de coulée. Il se forme un précipité blanc de bromhydrate de pyridine.

Après avoir laissé sous agitation durant une heure environ, on ajoute la quantité suffisante d'eau nécessaire à la dissolution du précipité de bromhydrate de pyridine. Après traitement du milieu réactionnel (décantations, extractions, etc) et distillation sous pression réduite, on recueille 191,0 g (105 °C/0,5 mm Hg) d'un produit de pureté 97 %, soit un rendement en produit pur de 75,7% (calculé sur les deux étapes, à partir de la fluoro-2-acroléine).

Exemple 9

Formation du dibromo-2,3-fluoro-2-propionate de méthyle

On opère de façon identique à celle décrite en 8B), en remplaçant le phénol par du méthanol.

On a ainsi obtenu le dibromo-2,3-fluoro-2-propionate de méthyle avec un rendement de l'ordre de 70 % calculé sur les deux étapes, à partir de la fluoro-2acroléine mise en œuvre.

Caractérisation :
Eb = 58-60 °C/1,5 mm Hg
RMN$^1$H = $-OCH_3$ = motif centré à 3,93 ppm
$-CH_2-$ = motif à 4 pics étalés de 4,49 à 4,04 ppm
RMN$^{19}$F = $CDCl_3/C_6F_6$ = motif centré à 4,39 ppm.

Exemple 10

Formation du dibromo-2,3-fluoro-2-propionate d'isopropyle

On opère dans un réacteur de 4 litres muni d'un agitateur mécanique, une ampoule de coulée et un réfrigérant. On introduit 3 690 g de $CCl_4$ et 165 g (2,75 moles) d'alcool isopropylique. On maintient à une température comprise entre — 5 et 0 °C et on coule 227,8 g de pyridine (2,88 moles).

Puis on ajoute du bromure de dibromo-2,3-fluoro-2-propanoyle, soit 1 172,6 g de bromure d'acide brut correspondant à la bromation de 2,75 moles de fluoro-2-acroléine.

Il y a formation d'un précipité de bromhydrate. On laisse l'agitation durant environ 24 heures jusqu'à ce que l'analyse montre la disparition du bromure d'acide.

On ajoute 170 ml d'eau. Après décantation, lavage de la phase organique par deux fois avec 150 ml $H_2O$, extraction des phases aqueuses par $CCl_4$, évaporation des solvants, on recueille, par distillation sous pression réduite (58 °C sous 1 mm Hg) : 663 g d'ester dibromé, pureté 97,1 %, soit un rendement en produit pur de 80,2 %.

Exemple 11

Formation du fluoro-2-acrylate de phényle

Dans un ballon d'un litre inerté à l'argon, muni d'une agitation et d'un réfrigérant, on introduit 110 g de dibromo-2,3-fluoro-2-propanolate de phényle (soit 0,337 mole) en solution dans 350 ml d'éther isopropylique, puis 65 g de zinc en poudre.

L'ensemble est maintenu à reflux durant 4 heures. Après retour à température ambiante, le milieu réactionnel est filtré. Après élimination des solvants, une distillation bulbe à bulbe permet de recueillir 44 g de fluoro-2-acrylate de phényle (0,264 mole), soit un rendement de l'ordre de 78,6 %.

Caractérisation :
Eb = 52-54 °C/0,5 mm Hg
RMN$^1$H = protons vinyliques : motif centré de 5,31 à 6,11 ppm
-phényl : motif centré à 7,27 ppm
RMN$^{19}$F = $CDCl_3/C_6F_6$ : motif centré à 47,65 ppm.

Exemple 12

Formation du fluoro-2-acrylate d'isopropyle

Dans un réacteur de 4 litres muni d'un agitateur mécanique, d'un réfrigérant et d'une ampoule de coulée, on introduit 2 litres d'éther isopropylique, 160 g de zinc en poudre (2,44 moles) et une pointe de spatule d'hydroquinone.

L'ensemble est porté à reflux tandis que l'on coule 586 g d'ester dibromé à 97 %, soit 1,95 mole.

Après refroidissement, filtration, lavage du précipité à l'éther isopropylique, lavage de la phase organique à l'eau pour éliminer les sels de zinc, on recueille par distillation 220,7 g de fluoroacrylate d'isopropyle à 99,4 % de pureté, soit un rendement de l'ordre de 85 %.

Il va de soi que la présente invention n'a été décrite qu'à titre purement explicatif et nullement limitatif et que toute modification utile pourra y être apportée sans sortir de son cadre.

**Revendications**

1. Produits industriels nouveaux répondant à la formule :

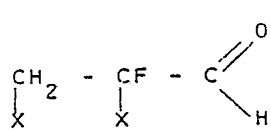

dans laquelle X = Cl ou Br.

2. Procédé d'obtention des produits selon la revendication 1, caractérisé en ce qu'il consiste à halogéner la fluoro-2-acroléine par addition directe de l'halogène $X_2$.

3. Procédé selon la revendication 2, caractérisé en ce que ladite fluoro-2-acroléine est obtenue par hydrolyse d'acétals de formule :

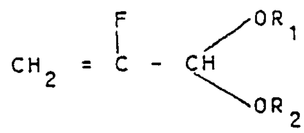

dans laquelle $R_1$ et $R_2$ représentent des groupes alkyles identiques ou différents ayant de 1 à 8 atomes de carbone, les acétals étant obtenus par l'ouverture dans des conditions douces et contrôlables, de cycles d'alcoxy-1-halogéno-2-fluoro-2-cyclopropanes de formule :

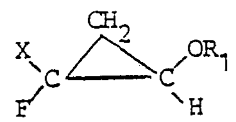

où $R_1$ est un alkyle en $C_1$ à $C_8$ et X est du chlore ou du brome, cette ouverture se faisant par solvolyse en milieu alcoolique mettant en œuvre des alcools $R_2OH$ avec $R_2$ = alkyle en $C_1$ à $C_8$ et en présence d'une base.

4. Procédé selon la revendication 3, caractérisé en ce que la base est choisie parmi la pyridine, la triéthanolamine et la soude.

5. Procédé selon la revendication 3, caractérisé en ce que l'alcoxy-1-halogéno-2-fluoro-2-cyclopropane est obtenu à partir d'un éther d'énol de formule :

$$CH_2 = CH - OR_1$$

avec $R_1$ = alkyle en $C_1$ à $C_8$.

6. Procédé selon la revendication 5, caractérisé en ce que l'on additionne un halogénofluorocarbène et plus particulièrement le chlorofluorocarbène : CFCl sur ledit éther d'énol.

7. Procédé selon la revendication 6, caractérisé en ce que ledit chlorofluorocarbène est obtenu in situ à partir de $CHFCl_2$ (« Fréon 21 ») en présence d'une base et d'un agent de transfert de phase.

8. Procédé selon la revendication 7, caractérisé en ce que ladite base est NaOH ou KOH.

9. Procédé selon la revendication 7, caractérisé en ce que ledit agent de transfert de phase est le chlorure de triéthylbenzylammonium ou le bromure de tétraéthylammonium.

10. Produits répondant à la formule :

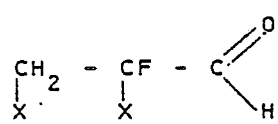

dans laquelle X = Cl ou Br

convenant pour être appliqués dans un procédé de fabrication d'un fluoroacrylate d'alkyle ou d'aryle de formule :

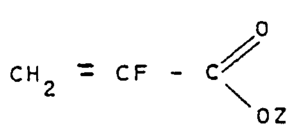

dans laquelle Z est un groupement alkyle ou aryle comprenant le stade d'obtention d'un halogénure de dihalogéno-2,3-fluoro-2-propanoyle de formule :

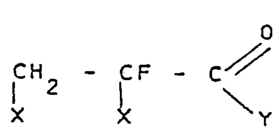

dans laquelle X et Y, identiques ou différents sont du Cl ou du Br par halogénation par un agent choisi parmi le chlore, le brome et la N-bromo-succinimide desdits produits selon la revendication 1 suivi du stade d'obtention d'un ester de formule :

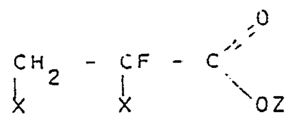

où Z a la signification ci-dessus, suivi du stade de déshalogénation pour obtenir ledit fluoroacrylate d'alkyle ou d'aryle.

11. Produit répondant à la formule :

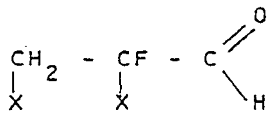

dans laquelle X = Br, convenant pour être appliqué dans un procédé de fabrication d'un fluoroacrylate d'alkyle ou d'aryle de formule :

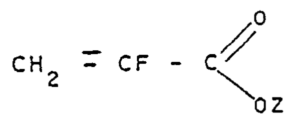

dans laquelle Z est un groupement alkyle ou aryle selon la revendication 10, dans lequel ledit halogénure de dihalogéno-2,3-fluoro-2-propanoyle est le composé nouveau de formule :

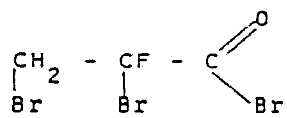

12. Produits répondant à la formule :

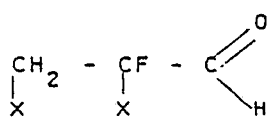

dans laquelle X = Cl ou Br, convenant pour être appliqués dans un procédé selon la revendication 10, dans lequel leur halogénation est réalisée par le brome sous radiation ultraviolette.

13. Produits répondant à la formule :

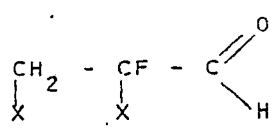

dans laquelle X = Cl ou Br, convenant pour être appliqués dans un procédé selon la revendication 10, dans lequel leur halogénation par la N-bromo-succinimide est réalisée en présence d'initiateurs de radicaux libres.

**Claims**

1. Novel industrial products complying with the formula :

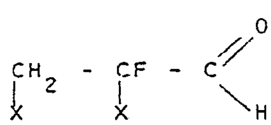

in which X =Cl or Br.

2. A process for preparing products according to Claim 1, characterized in that it consists of halogenating 2-fluoroacrolein by direct addition of halogen $X_2$.

3. A process according to Claim 2, characterized in that the said 2-fluoroacrolein is obtained by

hydrolysis of acetals having the formula :

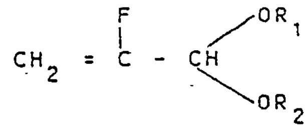

in which $R_1$ and $R_2$ represent identical or different alkyl groups having 1 to 8 carbon atoms, the acetals being obtained by opening under mild and controllable conditions the rings of 1-alkoxy-2-halogen-2-fluorocyclopropanes having the formula :

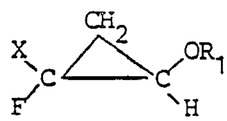

where $R_1$ is a $C_1$ to $C_8$ alkyl group and X is chlorine or bromine, this opening being performed by solvolysis in an alcoholic medium employing alcohols $R_2OH$, where $R_2$ is a $C_1$ to $C_8$ alkyl group, in the presence of a base.

4. A process according to Claim 3, characterized in that the base is chosen from pyridine, triethanolamine or soda.

5. A process according to Claim 3, characterized in that the 1-alkoxy-2-halogen-2-fluoro-cyclopropane is obtained from an ether of an enol having the formula :

$$CH_2 = CH — OR_1$$

where $R_1 = C_1$ to $C_8$ alkyl.

6. A process according to Claim 5, characterized in that a halogenfluorocarbene, and more especially chlorofluorocarbene : CFCl, is added to the said enol ether.

7. A process according to Claim 6, characterized in that the said chlorofluorocarbene is prepared in situ from $CHFCl_2$ (« Freon 21 ») in the presence of a base and a phase transfer agent.

8. A process according to Claim 7, characterized in that the said base is NaOH or KOH.

9. A process according to Claim 7, characterized in that the said phase transfer agent is triethylbenzylammonium chloride or tetraethylammonium bromide.

10. Products complying with the formula :

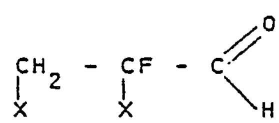

in which X = Cl or Br,
suitable for employment in a process for manufacturing an alkyl or aryl fluoracrylate having the formula :

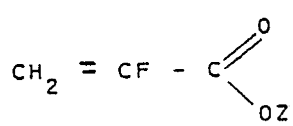

in which Z is an alkyl or aryl group, comprising the stage of preparing a halide of 2,3-dihalogen-2-fluoropropionic acid having the formula :

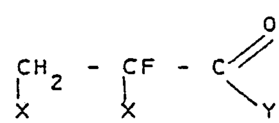

in which X and Y are Cl or Br, identical or different, by halogenation of the said products according to Claim 1 using an agent chosen from chlorine, bromine and N-bromo-succinimide, followed by the stage of preparing an ester having the formula :

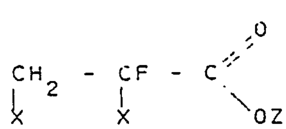

where Z has the meaning given above, followed by a dehalogenation stage to obtain the said alkyl or aryl fluoracrylate.

11. A product complying with the formula :

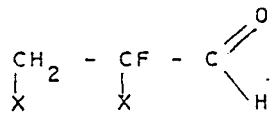

in which X = Br, suitable for employment in a process for manufacturing an alkyl or aryl fluoracrylate having the formula :

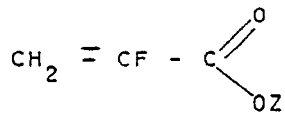

in which Z is an alkyl or aryl group according to Claim 10, in which the said halide of 2,3-dihalogen-2-fluoropropionic acid is the new compound having the formula :

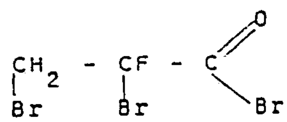

12. Products complying with the formula :

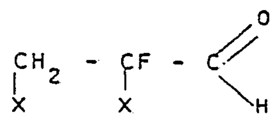

in which X = Cl or Br, suitable to be employed in a process according to Claim 10, in which their halogenation is performed by bromine using ultraviolet radiation.

13. Products complying with the formula :

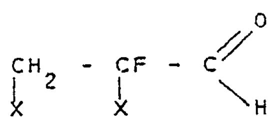

in which X = Cl or Br, suitable to be employed in a process according to Claim 10, in which their halogenation by N-bromo-succinimide is performed in the presence of initiators of free radicals.

**Patentansprüche**

1. Neue gewerblich anwendbare Erzeugnisse, die der Formel :

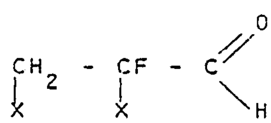

entsprechen, worin X = Cl oder Br ist.

2. Verfahren zum Herstellen der Erzeugnisse nach Anspruch 1, dadurch gekennzeichnet, daß es das Halogenieren von 2-Fluoracrolein durch direktes Zugeben des Halogens $X_2$ umfaßt.

3. Verfahren nach Anspruch 2, dadurch gekennzeichnet, daß das genannte 2-Fluoracrolein durch Hydrolyse der Acetale mit der Formel :

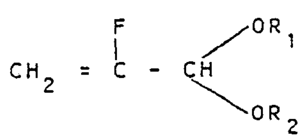

erhalten wurde, worin $R_1$ und $R_2$ identische oder unterschiedliche Alkylgruppen bedeuten, die 1 bis 8 Kohlenstoffatome besitzen, wobei die Acetale durch die Ringöffnung von 1-Alkoxy-2-halogen-2-fluor-2-cyclopropanen mit der Formel :

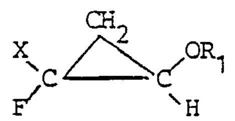

unter milden und kontrollierten Bedingungen erhalten wurden, worin $R_1$ ein Alkyl von $C_1$ bis $C_8$ ist und X Chlor oder Brom ist, und wobei diese Öffnung durch Solvolyse in alkoholischem Milieu unter Verwendung von Alkoholen $R_2OH$ mit $R_2$ = Alkyl von $C_1$ bis $C_8$ und in Gegenwart einer Base ausgeführt wird.

4. Verfahren nach Anspruch 3, dadurch gekennzeichnet, daß die Base unter Pyridin, Triethanolamin und Soda ausgewählt ist.

5. Verfahren nach Anspruch 3, dadurch gekennzeichnet, daß das 1-Alkoxy-2-halogen-2-fluor-cyclopropan ausgehend von einem Enolether mit der Formel :

$$CH_2 = CH — OR_1$$

mit $R_1$ = Alkyl von $C_1$ bis $C_8$ erhalten wurde.

6. Verfahren nach Anspruch 5, dadurch gekennzeichnet, daß man dem genannten Enolether ein Halogenfluorcarben und insbesondere Chlorfluorcarben : CFCl zusetzt.

7. Verfahren nach Anspruch 6, dadurch gekennzeichnet, daß das genannte Chlorfluorcarben ausgehend von $CHFCl_2$ (« Freon 21 ») in situ in Gegenwart einer Base und eines Phasentransferreagens erhalten wurde.

8. Verfahren nach Anspruch 7, dadurch gekennzeichnet, daß die genannte Base NaOH oder KOH ist.

9. Verfahren nach Anspruch 7, dadurch gekennzeichnet, daß das genannte Phasentransferreagens Triethylbenzylammoniumchlorid oder Tetraethylammoniumbromid ist.

10. Erzeugnisse gemäß der Formel :

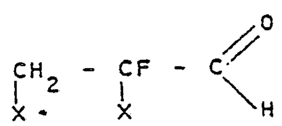

worin X = Cl oder Br ist, die sich für die Verwendung in einem Verfahren zum Herstellen eines Alkyl- oder Arylfluoracrylats mit der Formel

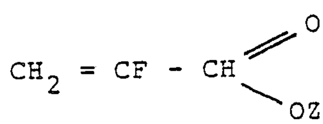

worin Z eine Alkyl- oder Arylgruppe ist, eignen, welches die Stufe der Herstellung eines 2,3-Dihalogen-2-fluorpropionsäurehalogenids mit der Formel

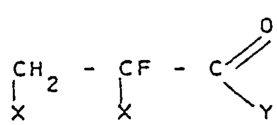

worin X und Y identisch oder unterschiedlich sind und Cl oder Br bedeuten, durch Halogenieren der genannten Erzeugnisse nach Anspruch 1 mit einem Reagens, das unter Chlor, Brom und N-Bromsuccinimid ausgewählt ist, gefolgt von der Stufe der Gewinnung eines Esters mit der Formel :

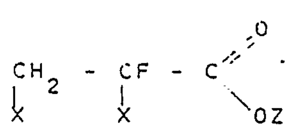

worin Z die oben angegebene Bedeutung besitzt, gefolgt von der Stufe der Dehalogenierung zur Gewinnung des genannten Alkyl- oder Arylfluoracrylats umfaßt.

11. Erzeugnis gemäß der Formel

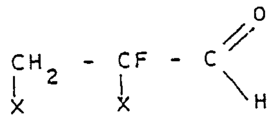

worin X = Br ist, das sich für die Verwendung in einem Verfahren zum Herstellen eines Alkyl- oder Arylfluoracrylats mit der Formel

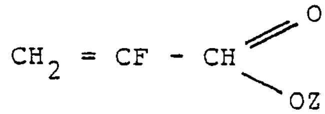

eignet, worin Z eine Alkyl- oder Arylgruppe nach Anspruch 10 ist, in welchem das genannte 2,3-Dihalogen-2-fluorpropionsäurehalogenid die neue Verbindung mit der Formel

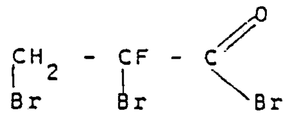

ist.

12. Erzeugnisse gemäß der Formel

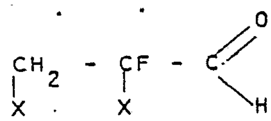

worin X = Cl oder Br ist, die sich zur Verwendung in einem Verfahren nach Anspruch 10 eignen, in welchem deren Halogenierung durch Brom unter ultravioletter Bestrahlung ausgeführt wird.

13. Erzeugnisse gemäß der Formel

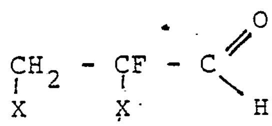

worin X = Cl oder Br ist, die sich zur Verwendung in einem Verfahren nach Anspruch 10 eignen, in welchem deren Halogenierung durch N-Bromsuccinimid in Gegenwart von Initiatoren für freie Radikale durchgeführt wird.